# EUROPÄISCHE PATENTANMELDUNG

(11) **EP 1 323 698 A2**
(43) Veröffentlichungstag der Anmeldung: **02.07.2003**
(21) Anmeldenummer: 02028037.6
(22) Anmeldetag: 16.12.2002
(51) Int. Cl.: C07C 29/38, C07C 31/22

(54) **Verfahren zur Herstellung von Trimethylolpropan**

(30) Priorität: 27.12.2001 DE 10164264
(71) Anmelder: Bayer Aktiengesellschaft, 51368 Leverkusen (DE)
(72) Erfinder: Müller, Dirk, Dr., 51467 Bergisch Gladbach (DE); Wagner, Paul, Dr., 40597 Düsseldorf (DE); Schwegler, Brian, 3930 Visp (CH); Notheis, Ulrich, Dr., 41439 Dormagen (DE); Armbrust, Ralph, Dr., 41539 Dormagen (DE); Heinz, Hans-Detlef, Dr., 51373 Leverkusen (DE); Wagner, Alexander, Dr., 47829 Krefeld (DE)

(57) **Zusammenfassung**

Die vorliegende Erfindung betrifft ein Verfahren zur Herstellung von Trimethylolpropan mit geringer APHA-Farbzahl.

## Beschreibung

Die vorliegende Erfindung betrifft ein Verfahren zur Herstellung von Trimethylolpropan mit geringer APHA-Farbzahl.

Trimethylolpropan, im Folgenden TMP genannt, ist ein dreiwertiger Alkohol von hoher industrieller Bedeutung, der beispielsweise Verwendung im Bereich der Herstellung von Lackharzen, Pulverlacken, Schaumstoffen und Polyestern findet.

Üblicherweise wird TMP aus n-Butyraldehyd und Formaldehyd hergestellt. Durch basenkatalysierte Aldolreaktionen wird dabei im 1. Reaktionsschritt zunächst aus n-Butyraldehyd und zwei Äquivalenten Formaldehyd 2,2-Dimethylolbutyraldehyd erzeugt.

Dieses kann dann im 2. Reaktionsschritt beispielsweise durch Umsetzung mit weiterem Formaldehyd und Base in einer sogenannten Cannizzaro-Reaktion in TMP-haltige Reaktionsmischungen überführt werden, wobei als weiteres Reaktionsprodukt auch das Formiat der eingesetzten Base anfällt.

Die Reaktionsschritte 1 und 2 können entweder separat oder in einem Arbeitsschritt durchgeführt werden. Sowohl für den basenkatalysierten Reaktionsschritt 1 als auch für den in Bezug auf die Basenmenge stöchiometrischen Reaktionsschritt 2 können gegebenenfalls unabhängig voneinander als Basen beispielsweise Alkali- oder Erdalkalimetallhydroxide oder -carbonate oder tertiäre Amine eingesetzt werden.

Wird für den separierten 2. Reaktionsschritt oder die in einem Arbeitschritt durchgeführten Reaktionsschritte 1 und 2 z. B. ein tertiäres Amin eingesetzt, spricht man vom organischen Cannizzaro-Verfahren, bei Einsatz von anorganischen Basen wie z.B. Alkali- oder Erdalkalimetallhydroxiden oder -carbonaten dementsprechend von einem anorganischen Cannizzaro-Verfahren. Die unterschiedlichen physikalischen und chemischen Eigenschaften der als weiteres Reaktionsprodukt auftretenden Ammoniumformiate bzw. anorganischen Formiate erfordern sehr unterschiedliche Aufarbeitungsmethoden für die TMP-haltigen Reaktionsmischungen.

Das anorganische Cannizzaro-Verfahren besitzt den Vorteil, dass das TMP in guter Reinheit anfällt und bei Verwendung z.B. von Calciumhydroxid als Base als Nebenprodukt Calciumformiat anfällt, das seinerseits zum Beispiel als Zusatzstoff für die Tierernährung, in der Baustoffindustrie, als Hilfsstoff in der Lederindustrie, als Hilfsmittel bei der Herstellung von Hochglanzpapieren, zur Behandlung von Waschwässern und bei der Rauchgasentschwefelung verwendet werden kann.

Die durch Cannizzaro-Verfahren erhältlichen TMP-haltigen Reaktionsmischungen weisen in der Regel eine sehr starke Färbung auf, die durch Verunreinigungen verursacht wird. Diese Verfärbung, die üblicherweise durch eine Farbzahl nach APHA (American Public Health Organization) oder Hazen angegeben werden kann, stört bei einigen Verwendungen, weshalb die Aufarbeitung üblicherweise aus Reinigungsoperationen wie z.B. Säurebehandlungen, Extraktionen und/oder mehrstufigen Destillationen besteht. Solche mehrstufigen Destillationen benötigen in der Regel teure, platzraubende und apparativ aufwändige Kolonnenanordnungen und sind daher wirtschaftlich wenig attraktiv.

Für die Aufarbeitung von TMP-haltigen Reaktionsmischungen aus anorganischen Cannizzaro-Verfahren sind verschiedene Techniken bekannt.

So ist beispielsweise in DD-P-45 078 ein Verfahren zur Aufarbeitung beschrieben, in dem aus anorganischen Cannizzaro-Verfahren erhaltene TMP-haltige Reaktionsmischungen mit sekundären cycloaliphatischen Alkoholen, die mit Wasser Azeotrope bilden, versetzt werden, dann Wasser zusammen mit diesem Alkohol azeotrop abdestilliert wird und die ausgefallenen Alkali- bzw. Erdalkaliformiate durch Filtration abgetrennt werden. Nach Abdestillieren des überschüssigen Alkohols wird das erhaltene rohe TMP zur weiteren Reinigung destilliert.

In der DD-Patentschrift 287 251 ist ein Verfahren zur Abtrennung von sogenannten Hochsiedern von TMP beschrieben. Hochsieder sind zum Beispiel Reaktionsfolgeprodukte von TMP, wie insbesondere Formale, die einen höheren Siedepunkt als TMP besitzen und sich daher bei der Vakuumdestillation von rohem TMP im Destillationsrückstand anreichern. Im beschriebenen Verfahren werden durch Zusatz von 0,02 bis 0,05 kg Säure/kg viele der Hochsieder zumindest teilweise wieder in TMP überführt, was zu einer Ausbeutesteigerung führen soll.

Auch die GB 1 290 036 beschreibt ein Verfahren zur Zersetzung von Hochsiedern in TMP-haltigen Reaktionsmischungen, die nach dem anorganischen Cannizzaro-Verfahren erhalten wurden. Dabei werden durch Zugabe von Kationenaustauscherharzen und Erhitzen auf 50°C bis 140°C gegebenenfalls vorhandene Formale, die einen ähnlichen Siedepunkt wie TMP haben und beim Siedepunkt von TMP zur Zersetzung neigen, in Produkte mit anderen Siedepunkten überführt, die leicht destillativ abtrennbar sind.

Die US 3,097,245 beschreibt ein Verfahren zur Herstellung von TMP mit einer APHA-Farbzahl zwischen 50 und 200. Diese Farbzahl wird durch Begrenzung der Reaktionszeit auf unter 7 Stunden, Acidifizierung des Reaktionsgemisches auf einen pH-Wert von unter 7 und Beschränkung der Konzentrationen der Ausgangsverbindungen auf Werte zwischen 5 und 20 Gew.-% erreicht. Weiterhin schließt sich an die Reaktion die Behandlung der erhaltenen Lösung mit Kationenaustauscherharzen und stark basischen quarternären Ammonium-Anionenaustauschern an.

Allen genannten Verfahren ist gemeinsam, dass chemische Behandlungsmethoden durchgeführt werden müssen, die sowohl die Ökobilanz als auch die wirtschaftliche Herstellbarkeit des Produktes verschlechtern oder einen erheblichen apparativen und damit finanziellen Aufwand erfordern, um Produkte mit einer akzeptablen Farbzahl zu erzeugen.

Es bestand daher das Bedürfnis, ein effizientes Verfahren bereitzustellen, das es ermöglicht, aus nach dem anorganischen Cannizzaro-Verfahren hergestellten TMP-haltigen Reaktionsmischungen reines TMP in hoher Ausbeute und einer möglichst geringen APHA-Farbzahl, d. h. von 20 oder darunter, zu gewinnen.

Es wurde nun ein Verfahren zur Herstellung von TMP mit einer möglichst geringen APHA-Farbzahl gefunden, das dadurch gekennzeichnet ist, dass
a) n-Butyraldehyd und/oder 2,2-Dimethylolbutyraldehyd mit Formaldehyd in Gegenwart von anorganischer Base zu TMP-haltigen Reaktionsmischungen umgesetzt wird,
b) aus der in a) erhaltenen TMP-haltigen Reaktionsmischung zur Gewinnung von rohem TMP Wasser und anorganische Salze zumindest teilweise entfernt werden und
c) das aus b) gewonnene rohe TMP, gegebenenfalls nach zumindest teilweiser Abtrennung von Schwersiedern und Nichtsiedern durch Destillation in eine oder mehrere Leichtsiederfraktionen, eine oder mehrere überwiegend TMP-haltige Mittelsiederfraktionen und eine oder mehrere Schwersieder- und/oder Nichtsiederfraktionen getrennt wird.

Es sei darauf hingewiesen, dass vom Rahmen der Erfindung auch beliebige Kombinationen der im Folgenden genannten Vorzugsbereiche mitumfasst sind.

Schritt a) des erfindungsgemäßen Verfahrens kann beispielsweise gemäß oder analog zu DE-OS 11 54 080, US 3 183 274 oder WO 01/51438 durchgeführt werden.

Als anorganische Basen können beispielsweise Alkali- oder Erdalkalimetallhydroxide und/oder -carbonate eingesetzt werden. Bevorzugt sind Natriumhydroxid, Kaliumhydroxid, Calciumhydroxid, Bariumhydroxid, Natriumcarbonat und Calciumcarbonat oder Mischungen davon, besonders bevorzugt sind Natriumhydroxid und Calciumhydroxid oder Mischungen davon, ganz besonders bevorzugt ist Calciumhydroxid.

Formaldehyd kann beispielsweise und bevorzugt in Form einer wässrigen Lösung mit einem Gehalt von etwa 1 bis 55 Gew.-% Formaldehyd, besonders bevorzugt mit einem Gehalt von 20 bis 40 Gew.-% Formaldehyd eingesetzt werden.

Weiterhin ist bevorzugt, Formaldehyd einzusetzen, der weniger als 10 mol-%, besonders bevorzugt 0 bis 6 mol-% und ganz besonders bevorzugt 0 bis 4 mol-% Methanol bezogen auf Formaldehyd enthält.

In einer bevorzugten Ausführungsform wird n-Butyraldehyd mit Formaldehyd in Gegenwart von anorganischer Base zu TMP-haltigen Reaktionsmischungen umgesetzt. Das molare Verhältnis von Formaldehyd zu n-Butyraldehyd kann dabei beispielsweise 2:1 bis 10:1, bevorzugt 3:1 bis 4:1 und besonders bevorzugt 3,1:1 bis 3,4:1 betragen. Der Einsatz größerer molarer Mengen Formaldehyd als 10:1 ist möglich, aber unwirtschaftlich.

Das molare Verhältnis von Base zu n-Butyraldehyd kann beispielsweise 1,0 bis 1,5, bevorzugt 1,01 bis 1,4 und besonders bevorzugt 1,02 bis 1,3 betragen. Der Einsatz größerer molarer Mengen an Base kann, insbesondere bei höheren Reaktionstemperaturen, zu Ausbeuteverlusten durch Nebenreaktionen führen und ist daher unwirtschaftlich. Die Wertigkeit der eingesetzten Base ist dabei zu berücksichtigen.

Der Druck bei der Umsetzung ist unkritisch; die Reaktion kann beispielsweise bei Atmosphärendruck, Drücken zwischen 0,5 bar und Atmosphärendruck oder Drücken zwischen Atmosphärendruck und 10 bar ausgeführt werden, bevorzugt wird die Reaktion bei Atmosphärendruck ausgeführt. Die Durchführung der Reaktion bei höheren Drücken ist möglich, aber ökonomisch nicht sinnvoll.

Die Temperatur bei der Umsetzung kann beispielsweise 0°C bis 100°C betragen, bevorzugt sind Temperaturen zwischen 10°C und 90°C, besonders bevorzugt zwischen 20 und 70°C. Höhere Temperaturen und längere Reaktionszeiten können zu einem erhöhten Anteil unerwünschter Nebenreaktionen und zu stärker verfärbten Produkten führen.

Die Reaktionszeit für die Umsetzung kann beispielsweise 0,1 bis 24 Stunden betragen, bevorzugt sind 0,2 bis 4 Stunden. Dabei kann die im Einzelfall bevorzugte Reaktionszeit in üblicher Weise vom Fachmann leicht bestimmt werden, beispielsweise durch Verfolgen des Reaktionsverlaufes mit analytischen Methoden.

Gemäß Schritt a) kann eine TMP-haltige Reaktionsmischung erhalten werden. Diese TMP-haltige Reaktionsmischung enthält üblicherweise Wasser und anorganische Formiate, gegebenenfalls überschüssiges Formaldehyd, gegebenenfalls überschüssige Base sowie Produkte, die beispielsweise aus Eliminationsreaktionen, Zersetzungsund Folgereaktionen stammen können. Ohne Anspruch auf Vollständigkeit können das beispielsweise sein:

Lineare und verzweigte Alkohole, wie beispielsweise 2-Ethyl-1,3-propandiol und 2-{[2,2-Bis(hydroxymethyl)butoxy]methyl}-2-ethyl-1,3-propandiol (Di-TMP); Formale, wie beispielsweise 2-Ethyl-2-[(methoxymethoxy)methyl]-1,3-propandiol oder 2-({[2,2-bis(hydroxymethyl)butoxy]methoxy}methyl)-2-ethyl-1,3-propandiol (Bisformal); Ester, wie TMP-Monoformiat und TMP-Monobutyrat und Ether, wie 2-Ethyl-2-(methoxymethyl)-1,3-propandiol.

Gemäß Schritt b) des erfindungsgemäßen Verfahrens werden aus der TMP-haltigen Reaktionsmischung Wasser und anorganische Salze entfernt.

Die Entfernung von Wasser und anorganischen Salzen kann beispielsweise dadurch erfolgen, dass die TMP-haltige Reaktionsmischung mit einem mit Wasser nicht oder nur wenig mischbaren Lösungsmittel einmal oder mehrmals extrahiert wird. Diese Extraktion kann beispielsweise gemäß oder analog zu WO 99/20586 oder US 5,603,835 durchgeführt werden.

Als Lösungsmittel für die Extraktion eignen sich dabei beispielsweise Ether, Aldehyde, Ketone, Alkohole oder Ester. Beispiele für geeignete Ether sind Methyl-tert.butylether, Di-n-propylether und Diglyme, für Aldehyde Butyraldehyd, für Ketone Methylisobutylketon oder Methylethylketon, für Alkohole 2-Ethylhexanol oder n-Octanol, als Ester können solche mit 2 bis 9 Kohlenstoffatomen wie beispielsweise Methylacetat oder Ethylacetat verwendet werden. Es können auch Mischungen solcher Lösungsmittel eingesetzt werden.

Anschließend kann das eingesetzte Lösungsmittel in an sich bekannter Weise beispielsweise durch Destillation entfernt werden.

Alternativ dazu und für das erfindungsgemäße Verfahren bevorzugt ist zunächst die Abtrennung von Wasser mittels Destillation und anschließend die Abtrennung von Feststoffen, die überwiegend anorganische Salze enthalten.

Anorganische Salze sind dabei im Wesentlichen anorganische Formiate und gegebenenfalls nicht umgesetzte anorganische Base.

Die Destillation kann nach an sich bekannten Methoden durchgeführt werden, bevorzugt ist der Einsatz eines oder mehrerer Verdampfer(s), besonders bevorzugt ist der Einsatz mehrstufiger Eindampfanlagen, bei denen die einzelnen Stufen bei verschiedenen Drücken und damit auch verschiedenen Temperaturen betrieben werden, um die Brüden einzelner oder mehrerer Stufen zur Beheizung weiterer Stufen zu nutzen und so den Energieaufwand gegenüber einstufigen Eindampfanlagen deutlich zu reduzieren.

Die Drücke bei den Destillationsschritten können beispielsweise im Bereich zwischen 20 mbar und 1 bar liegen. Bei diesen Drücken können sich in Abhängigkeit vom Wassergehalt des Gemisches beispielsweise Temperaturen im Bereich von 50 bis 120°C einstellen.

In einer bevorzugten Ausführungsform wird so lange destilliert, bis der Wassergehalt des Destillationsrückstandes weniger als 10 Gew.-%, bevorzugt weniger als 5 Gew.-%, und besonders bevorzugt weniger als 2 Gew.-% beträgt.

Die Abtrennung der gegebenenfalls ausgefallenen Feststoffe kann beispielsweise durch Filtration, Zentrifugation oder Sedimentation und Dekantieren erfolgen, wobei Zentrifugation sowie Sedimentation und Dekantieren bevorzugt sind.

Gegebenenfalls können die Schritte Destillation und nachfolgende Abtrennung gegebenenfalls ausgefallener Feststoffe wiederholt werden.

Wurden als Base calciumhaltige anorganische Basen wie z.B. Calciumhydroxid und/oder -carbonat eingesetzt, so enthält der ausgefallene Feststoff Calciumformiat, das gegebenenfalls nach Reinigungsoperationen den oben genannten Verwendungen zugeführt werden kann.

Auf beschriebene Weise erhält man aus der gemäß Schritt a) erhältlichen TMP-haltigen Reaktionsmischung in Schritt b) von Wasser und anorganischen Formiaten zumindest teilweise befreites, rohes TMP mit einem Gehalt von über 75 Gew.-%, bevorzugt über 80 Gew.-% TMP.

Das rohe TMP kann direkt in Schritt c) eingesetzt werden.

Alternativ dazu kann das gemäß Schritt b) erhaltene rohe TMP zunächst zumindest teilweise von Schwersiedern und Nichtsiedern befreit werden.

Im Rahmen der Erfindung sind unter Schwersiedern solche organischen Verbindungen zu verstehen, die einen Siedepunkt besitzen, der höher als der von TMP ist.

Beispielsweise, aber nicht abschließend, sind das Verbindungen wie 2-Ethyl-1,3-propandiol, Di-TMP und Bisformal.

Unter Nichtsiedern sind im Rahmen der Erfindung Verbindungen gemeint, die sich nicht oder irreversibel chemisch verändert destillieren lassen. Beispielsweise, aber nicht abschließend, sind das anorganische Salze und oligomere und polymere Verbindungen mit einer Molmasse von ca. 300 g/mol und darüber oder mehr als drei vom TMP oder Dimethylbutyraldehyd abgeleiteten Grundeinheiten.

Die zumindest teilweise Abtrennung von Schwersiedern und Nichtsiedern kann beispielsweise durch Destillation, bevorzugt durch Destillation mittels Verdampfer wie z.B. einem Dünnschichtverdampfer, erfolgen.

Der Destillationsrückstand, der die Schwersieder und gegebenenfalls vorhandene anorganische Salze enthält, kann beispielsweise verworfen, zur Heizdampferzeugung thermisch verwertet oder zur Gewinnung von Verbindungen wie zum Beispiel Di-TMP genützt werden.

Wird dieser sich an Schritt b) anschließende Verfahrensschritt durchgeführt, so erhält man ein vorgereinigtes, rohes TMP, das vorzugsweise einen Gehalt von über 80 Gew.-%, bevorzugt von über 90 Gew.-% und besonders bevorzugt von über 94 Gew.-% TMP aufweist.

Das vorgereinigte, rohe TMP besitzt in der Regel Farbzahlen von über 100 nach APHA.

Das vorgereinigte, rohe TMP oder das gemäß Schritt b) erhaltene rohe TMP wird in Schritt c) durch Destillation in eine oder mehrere Leichtsiederfraktionen, eine oder mehrere überwiegend TMP-haltige Mittelsiederfraktionen und eine oder mehrere Schwersiederfraktionen getrennt. Bevorzugt wird das vorgereinigte, rohe TMP oder das gemäß Schritt b) erhaltene rohe TMP in jeweils eine Leichtsiederfraktion, eine überwiegend TMP-haltige Mittelsiederfraktion und eine Schwersiederfraktion getrennt.

Im Rahmen der Erfindung sind unter Leichtsiedern solche Verbindungen zu verstehen, die einen Siedepunkt besitzen, der niedriger als der von TMP ist. Beispielsweise, aber nicht abschließend, sind das Reste von Wasser, Formaldehyd, 2-Ethyl-1,3-propandiol oder 2-Ethylacrolein.

Im Rahmen der Erfindung sind unter überwiegend TMP-haltigen Mittelsiederfraktionen solche Fraktionen zu verstehen, die einen Gehalt von über 90 Gew.-%, bevorzugt über 98 Gew.-% und besonders bevorzugt über 99 Gew.-% TMP enthalten und eine Farbzahl nach APHA von 50 oder weniger, bevorzugt 20 oder weniger, besonders bevorzugt 15 oder weniger und ganz besonders bevorzugt 10 oder weniger aufweisen.

Die erfindungsgemäße Destillation des rohen TMP oder des vorgereinigten, rohen TMP kann batchweise oder kontinuierlich erfolgen, wobei die kontinuierliche Destillation bevorzugt ist.

In einer besonders bevorzugten Ausführungsform des erfindungsgemäßen Verfahrens werden zur Destillation bevorzugt Destillationskolonnen eingesetzt, in denen eine oder mehrere Leichtsiederfraktionen und eine oder mehrere überwiegend TMP-haltige Mittelsiederfraktionen simultan entnommen werden können. Bevorzugt sind das solche Destillationskolonnen, die sich für die Seitenentnahme flüssiger und/oder gasförmiger Medien eignen, wie zum Beispiel Seitenstromkolonnen, Trennwandkolonnen oder thermisch gekoppelte Destillationskolonnen, wobei der Einsatz von Trennwandkolonnen im erfindungsgemäßen Verfahren weiter bevorzugt ist.

Geeignete Trennwandkolonnen bzw. thermisch gekoppelte Kolonnen sind z.B. in der EP-A 122 367, EP-A 0 126 288 und in der EP-A 0 133 510 aufgeführt. Insbesondere Trennwandkolonnen und thermisch gekoppelte Kolonnen bieten gegenüber der Anordnung von konventionellen Destillationskolonnen sowohl hinsichtlich des Energiebedarfs als auch der Investitionskosten enorme Vorteile.

Üblicherweise werden dabei über den Kolonnenkopf der Destillationsapparatur die Leichtsiederfraktionen entnommen, die farbgebende Komponenten enthalten können, und über Seitenabzüge die überwiegend TMP-haltigen Mittelsiederfraktionen. Als Destillationsrückstand verbleibt üblicherweise eine stark gefärbte Schwersiederfraktion, die gegebenenfalls noch Nichtsieder enthalten kann.

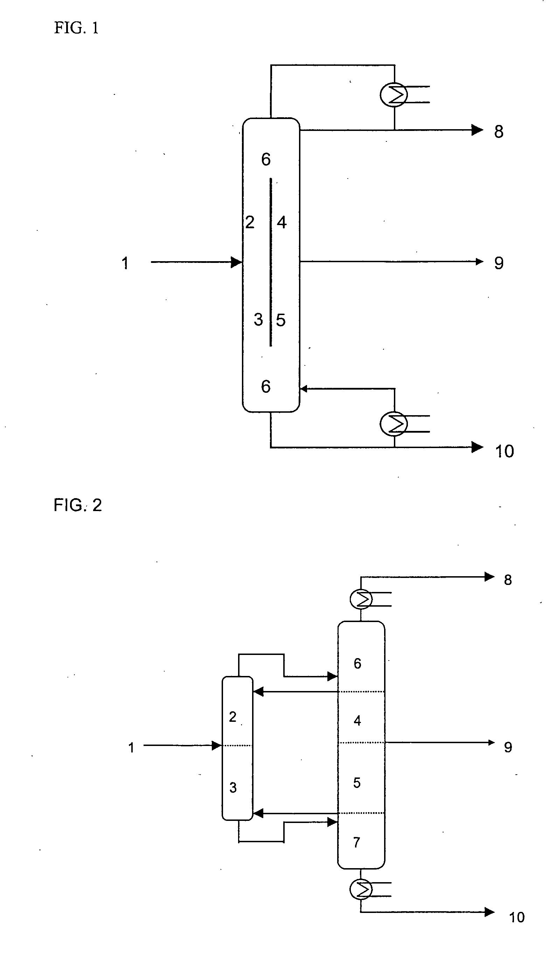

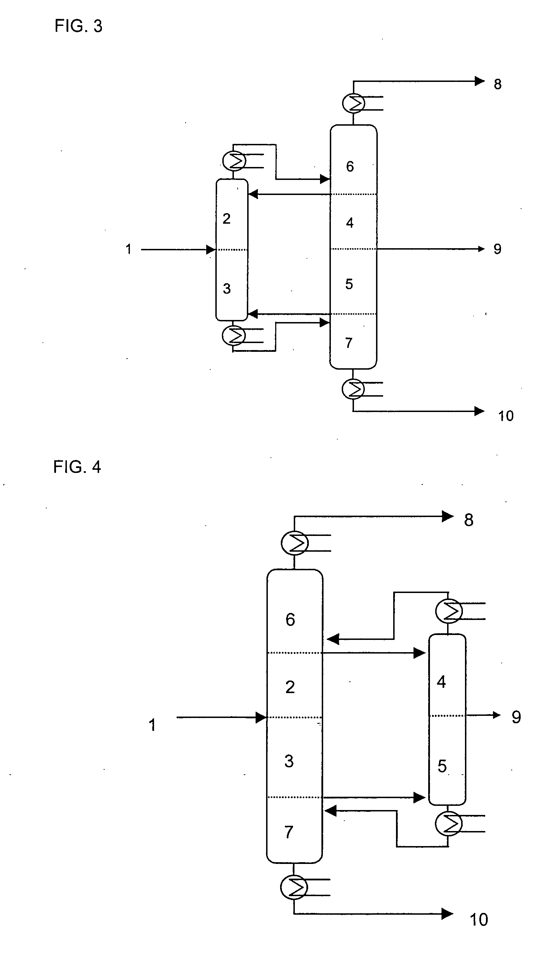

Anhand der Zeichnungen 1 bis 4 (Fig. 1, Fig. 2, Fig. 3, Fig. 4) werden im Folgenden die destillative Trennung des aus b) gewonnenen rohen TMP oder des vorgereinigten, rohen TMP beispielhaft erläutert, ohne den Gegenstand der Erfindung jedoch darauf zu beschränken.

Die Zeichnung 1 (Fig. 1) zeigt die vereinfachte Darstellung des Produktstroms der Trennung an einer Trennwandkolonne. Die gewählte Nummerierung gilt für die in Zeichnung 2, 3 und 4 dargestellten thermisch gekoppelten Destillationskolonnen analog.

Das rohe TMP oder das vorgereinigte, rohe TMP [1] wird über einen Zulauf der Kolonne zugeführt. Die Kolonne weist einen Zulaufteil [2], [3], einen Entnahmeteil [4], [5], einen oberen Kolonnenbereich (Kolonnenkopfbereich) [6] und einen unteren Kolonnenbereich auf, in dem sich üblicherweise der Destillationsrückstand (Sumpf) befindet [7].

Über den Kolonnenkopfbereich [6] wird die Leichtsiederfraktion 8 entnommen, über den Entnahmeteil [4], [5] die überwiegend TMP-haltige Mittelsiederfraktion [9] und über den unteren Kolonnenbereich [7] die Schwersiederfraktion [10], die entweder verworfen oder thermisch verwertet werden kann, z.B. zur Gewinnung von Heißdampf.

Die kontinuierliche Abtrennung der gegebenenfalls nichtsiederhaltigen Schwersiederfraktion [10] bietet insbesondere den Vorteil, dass die Ansammlung von schwer- und/oder nichtsiedenden und/oder stark verfärbten Komponenten verhindert wird, die durch thermische Belastung von rohem TMP oder vorgereinigtem, rohen TMP durch Nebenreaktionen entstehen können.

Die erfindungsgemäß einsetzbaren Trennwandkolonnen und thermisch gekoppelte Kolonnen können beispielsweise und bevorzugt als Füllkörper enthaltende oder als geordnete Packungen enthaltende Packungskolonnen oder als Bodenkolonnen ausgestaltet sein.

Typische Böden, Füllkörper und Packungen sind zum Beispiel in Henry Kister, Distillation Design, McGrawHill, 1992 beschrieben. Auch können Hochleistungspackungen, wie zum Beispiel die Packung MellapakPlus der'Firma Sulzer Chemtech, Winterthur/ Schweiz, eingesetzt werden.

Bei Einsatz von thermisch gekoppelten Kolonnen ist es vorteilhaft, sie so auszugestalten, dass ausschließlich flüssige Verbindungsströme zwischen den einzelnen Destillationskolonnen auftreten.

Bei Einsatz von Trennwandkolonnen oder thermisch gekoppelten Kolonnen, die als Bodenkolonnen ausgestaltet sind, kann die Anzahl der Böden beispielsweise 4 bis 150, bevorzugt 8 bis 70, besonders bevorzugt 10 bis 45 betragen. Die Böden von Trennwandkolonnen, die als Bodenkolonnen ausgestaltet sind, sind bevorzugt so angeordnet, dass die Trennwand in einem Winkel von ca. 80 bis 90° zu den entsprechenden Zulauf- und Ablaufschächten der Böden steht.

Bei Einsatz von Trennwandkolonnen oder thermisch gekoppelten Kolonnen, die als Packungskolonnen ausgestaltet sind, kann die Anzahl der theoretischen Trennstufen beispielsweise 5 bis 100, bevorzugt 8 bis 50, besonders bevorzugt 10 bis 35 betragen.

Ferner ist es auch möglich, die Kolonne sowohl mit Böden als auch mit Packungselementen auszustatten. Zum Beispiel können im Verstärkungsteil Packungen und im Abtriebsteil Böden eingesetzt werden.

Für das erfindungsgemäße Verfahren können insbesondere auch Trennwandkolonnen verwendet werden, über die am oberen und am unteren Ende der Trennwand flüssige oder gasförmige Proben kontinuierlich oder diskontinuierlich entnommen werden können. Solche Trennwandkolonnen wie auch die oben ausgeführten Kolonnentypen sind dem Fachmann hinlänglich bekannt.

Die erfindungsgemäße Destillation des rohen TMP oder des vorgereinigten, rohen TMP kann beispielsweise bei einer Temperatur von 150 bis 260°C, bevorzugt 180 bis 250°C und besonders bevorzugt 180 bis 235°C erfolgen und beispielsweise bei Drücken von 0,5 bis 500 mbar, bevorzugt 2 bis 100 mbar, besonders bevorzugt 2 bis 50 mbar und ganz besonders bevorzugt 5 bis 30 mbar durchgeführt werden.

Dabei bezieht sich die Temperaturangabe auf die im unteren Kolonnenbereich gemessene Temperatur des Sumpfes, die Druckangabe auf die im Kolonnenkopfbereich messbaren Werte.

Um die durch thermische Belastung des rohen TMP oder des vorgereinigten, rohen TMP möglichen Reaktionen, bei denen sich unerwünschte farbgebende und damit die Produktqualität verschlechternde Komponenten bilden können, zu minimieren, sind durchschnittliche Verweilzeiten in den Kolonnen von beispielsweise 2 Minuten bis 12 Stunden bevorzugt, 5 Minuten bis 2 Stunden besonders bevorzugt.

Die auf erfindungsgemäße Weise erhaltenen, überwiegend TMP-haltigen Mittelsiederfraktionen können erneut einer Reinigungsoperation, wie zum Beispiel einer erneuten Destillation, unterworfen werden, nötig ist dies jedoch nicht.

Das erfindungsgemäß hergestellte TMP eignet sich insbesondere zur Herstellung von Lackharzen, Pulverlacken, Schaumstoffen oder Polyestern.

Das erfindungsgemäße Verfahren besitzt den Vorteil, dass aus nach dem anorganischen Cannizzaro-Verfahren erhältliche TMP-haltige Reaktionsmischungen bei geringem apparativen Aufwand in höchst effizienter Weise TMP mit Reinheiten von über 99 % und Farbzahlen von unter 10 nach APHA erzielt werden können. Weiterhin stellt das Verfahren aufgrund des niedrigen Energieaufwandes für die erfindungsgemäße Destillation ökologisch und wirtschaftlich einen Fortschritt dar.

### Beispiele

### Beispiel 1

In einem thermostatisierten 1 L Doppelmantelrührbehälter mit mechanischem Rührer werden 400 g Wasser und 420 g einer 30%igen wässrigen Formaldehydlösung bei 25°C vorgelegt. Anschließend werden 54 g Calciumhydroxid zugegeben und innerhalb von einer Stunde 97 g Butyraldehyd zudosiert. In dieser Zeit wird die Temperatur auf 45°C angehoben. Anschließend wird 45 Minuten nachgerührt, dann auf 25°C abgekühlt und die Reaktionsmischung mit 85%iger Ameisensäure (Gew.-%) auf pH 6 eingestellt. Man erhält 971 g einer TMP-haltigen Reaktionsmischung mit einem TMP-Gehalt von15,1 Gew.-%.

Drei nach obiger Vorschrift hergestellte Ansätze werden vereinigt, auf dem Rotationsverdampfer im Vakuum eingeengt und über eine Drucknutsche filtriert. Auf der Nutsche verbleiben 287 g feuchter Rückstand, der nochmals mit 153 g Wasser angeschlämmt und abgesaugt wird. Filtrat und Waschflüssigkeit werden vereinigt und nochmals auf dem Rotationsverdampfer im Vakuum eingeengt. Auswaage: 521 g rohes TMP mit einem TMP-Gehalt von 80,5 Gew.-%

### Beispiel 2

1.032 g einer gemäß Beispiel 1 hergestellten TMP-Reaktionslösung mit einem TMP-Gehalt von 80,5 % werden aus einem vorgeheizten Tropftrichter mit einer Dosiergeschwindigkeit von 700 g pro Stunde in einen Dünnschichtverdampfer eindosiert. Der Dünnschichtverdampfer wird bei 215°C und 8 mbar betrieben. Hinter den Dünnschichtverdampfer sind zwei mit Trockeneis gekühlte Kühlfallen angeschlossen.

Man erhält 873 g als Kopfprodukt vorgereinigtes, rohes TMP mit einem TMP-Gehalt von 94,9 % (APHA-Farbzahl 148) und 93 g hochviskosen dunkelbraunen Sumpfablauf. Aus den Kühlfallen werden 37 g Leichtsieder isoliert.

### Beispiel 3

Vorgereinigtes, rohes TMP gemäß Beispiel 2 wurde in einer Destillationsapparatur, bestehend aus einem 1.000 mm hohen Destillationsabschnitt zur Aufkonzentrierung von Leichtsiedern (Verstärkungsteil), einem unterhalb des Verstärkungsteils angeordneten, 1.500 mm hohen und durch eine Trennwand geteilten mittleren Destillationsabschnitt sowie einem unterhalb des mittleren Destillationsabschnittes angeordneten, 1.000 mm hohen Destillationsabschnitt zur Aufkonzentrierung von Schwersiedern und Nichtsiedern (Abtriebsteil). Die Destillationsapparatur war ferner am oberen Ende der Kolonne mit einem Kondensator und am unteren Ende der Kolonne mit einem Verdampfer ausgestattet. Darüber hinaus war die Apparatur zur Vermeidung von Wärmeverlusten mit einem Heizmantel ausgerüstet, wobei sowohl innerhalb der Kolonne als auch im Heizmantel die Temperaturen gemessen wurden und der Heizmantel so geregelt wurde, dass sich in der Kolonne und im Heizmantel das gleiche Temperaturprofil über der Kolonnenhöhe einstellte.

Die destillative Trennung erfolgte bei einem Rücklaufverhältnis am Kopf der Kolonne von 48. Der Zulaufmengenstrom betrug 2,8 kg/h. Der Kopfdruck der Anlage betrug 75 mbar. Bei diesem Druck stellte sich eine Kopftemperatur von 152°C und eine Sumpftemperatur von 216°C ein. Mit dieser Fahrweise wurde TMP mit einem Gehalt von 99,5 Gew.-% und einer APHA-Farbzahl von 13 erhalten.

### Beispiel 4

Das rohe TMP gemäß Beispiel 2 wurde in der in Beispiel 3 beschriebenen Destillationsapparatur destillativ getrennt, wobei ein Kopfdruck von 15 mbar eingestellt wurde. Bei diesem Druck stellte sich eine Kopftemperatur von 125°C und eine Sumpftemperatur von 184°C ein. Das Rücklaufverhältnis am Kopf der Kolonne betrug wie im ersten Beispiel 48. Der Zulaufmengenstrom betrug 1,5 kg/h. Bei dieser Fahrweise wurde TMP mit einer Reinheit von 99,1 Gew.-% sowie einer APHA-Farbzahl von 8 erhalten.

### Beispiel 5 (Vergleichsbeispiel)

Vorgereinigtes, rohes TMP aus Beispiel 2 wurde in der Destillationsapparatur aus Beispiel 3 zweistufig getrennt, indem die Kolonne ohne Seitenabzug betrieben wurde. Dabei wurden im ersten Schritt Leichtsieder am Kopf der Kolonne entnommen und das Produkt TMP zusammen mit Schwersiedern und Nichtsiedern als Sumpfprodukt entnommen. Die Destillation wurde analog zu der Destillation in Beispiel 3 bei einem Kopfdruck von 75 mbar durchgeführt. Anschließend wurde das Sumpfprodukt des ersten Schrittes erneut der Kolonne zugeführt, um das Produkt TMP über Kopf zu destillieren und Schwersieder und Nichtsieder als Sumpfstrom zu entnehmen. Mit dieser Fahrweise wurde TMP mit einem Gehalt von 99,3 Gew.-% und einer APHA-Farbzahl von lediglich 22 erhalten.

## Patentansprüche

1. Verfahren zur Herstellung von Trimethylolpropan mit geringer APHA-Farbzahl, **dadurch gekennzeichnet, dass**
a) n-Butyraldehyd und/oder 2,2-Dimethylolbutyraldehyd mit Formaldehyd in Gegenwart von anorganischer Base zu Trimethylolpropan-haltigen Reaktionsmischungen umgesetzt wird,
b) aus der in a) erhaltenen Trimethylolpropan-haltigen Reaktionsmischung zur Gewinnung von rohem Trimethylolpropan Wasser und anorganische Salze zumindest teilweise entfernt werden und
c) das aus b) gewonnene rohe Trimethylolpropan durch Destillation in eine oder mehrere Leichtsiederfraktionen, eine oder mehrere überwiegend Trimethylolpropan-haltige Mittelsiederfraktionen und eine oder mehrere Schwersiederfraktionen getrennt wird.

2. Verfahren nach Anspruch 1, **dadurch gekennzeichnet, dass** im Schritt a) n-Butyraldehyd mit Formaldehyd in Gegenwart von anorganischer Base zu Trimethylolpropan-haltigen Reaktionsmischungen umgesetzt wird.

3. Verfahren nach einem oder mehreren der Ansprüche 1 bis 2, **dadurch gekennzeichnet, dass** im Schritt a) als anorganische Base Alkali- oder Erdalkalimetallhydroxide oder -carbonate oder Mischungen davon eingesetzt werden.

4. Verfahren nach einem oder mehreren der Ansprüche 1 bis 3, **dadurch gekennzeichnet, dass** im Schritt a) als anorganische Basen Natriumhydroxid, Calciumhydroxid oder Mischungen davon eingesetzt werden.

5. Verfahren nach einem oder mehreren der Ansprüche 1 bis 4, **dadurch gekennzeichnet, dass** im Schritt a) als anorganische Base Calciumhydroxid eingesetzt wird.

6. Verfahren nach einem oder mehreren der Ansprüche 1 bis 5, **dadurch gekennzeichnet, dass** im Schritt a) Formaldehyd in Form einer wässrigen Lösung mit einem Gehalt von 1 bis 55 Gew.-% eingesetzt wird.

7. Verfahren nach einem oder mehreren der Ansprüche 1 bis 6, **dadurch gekennzeichnet, dass** im Schritt a) Formaldehyd eingesetzt wird, der weniger als 10 mol-% Methanol enthält.

8. Verfahren nach einem oder mehreren der Ansprüche 1 bis 7, **dadurch gekennzeichnet, dass** im Schritt a) das molare Verhältnis von Formaldehyd zu n-Butyraldehyd 2:1 bis 10:1 beträgt.

9. Verfahren nach einem oder mehreren der Ansprüche 1 bis 8, **dadurch gekennzeichnet, dass** im Schritt a) das molare Verhältnis von Base zu n-Butyraldehyd 1,0 bis 1,4 beträgt.

10. Verfahren nach einem oder mehreren der Ansprüche 1 bis 9, **dadurch gekennzeichnet, dass** im Schritt a) der Druck bei der Reaktion 0,5 bis 10 bar beträgt.

11. Verfahren nach einem oder mehreren der Ansprüche 1 bis 10, **dadurch gekennzeichnet, dass** im Schritt a) die Temperatur bei der Reaktion 0 bis 100°C beträgt.

12. Verfahren nach einem oder mehreren der Ansprüche 1 bis 11, **dadurch gekennzeichnet, dass** im Schritt b) zunächst Wasser mittels Destillation entfernt wird und anschließend die Abtrennung von Feststoffen erfolgt.

13. Verfahren nach Anspruch 12, **dadurch gekennzeichnet, dass** im Schritt b) so lange destilliert wird, bis der Wassergehalt des Destillationsrückstandes weniger als 10 Gew.-% beträgt.

14. Verfahren nach einem oder mehreren der Ansprüche 12 bis 13, **dadurch gekennzeichnet, dass** die Abtrennung von Feststoffen durch Filtration, Zentrifugation oder Sedimentation und Dekantieren erfolgt.

15. Verfahren nach einem oder mehreren der Ansprüche 12 bis 14, **dadurch gekennzeichnet, dass** die Entfernung von Wasser mittels Destillation und die Abtrennung von Feststoffen einmal oder mehrfach wiederholt wird.

16. Verfahren nach einem oder mehreren der Ansprüche 1 bis 15, **dadurch gekennzeichnet, dass** das gemäß Schritt b) erhaltene rohe TMP zumindest teilweise von Schwersiedern und Nichtsiedern befreit wird, bevor es Schritt c) zugeführt wird.

17. Verfahren nach einem oder mehreren der Ansprüche 1 bis 16, **dadurch gekennzeichnet, dass** die Destillation in Schritt c) kontinuierlich betrieben wird.

18. Verfahren nach einem oder mehreren der Ansprüche 1 bis 17, **dadurch gekennzeichnet, dass** während der Destillation simultan eine oder mehrere Leichtsiederfraktionen und eine oder mehrere überwiegend Trimethylolpropan-haltige Mittelsiederfraktionen entnommen werden.

19. Verfahren nach einem oder mehreren der Ansprüche 1 bis 18, **dadurch gekennzeichnet, dass** während der Destillation simultan eine oder mehrere Leichtsiederfraktionen und eine oder mehrere überwiegend Trimethylolpropan-haltige Mittelsiederfraktionen und eine Schwersiederfraktion, die auch Nichtsieder enthalten kann, entnommen wird.

20. Verfahren nach einem oder mehreren der Ansprüche 1 bis 19, **dadurch gekennzeichnet, dass** für die Destillation in Schritt c) Seitenstromkolonnen, Trennwandkolonnen oder thermisch gekoppelte Destillationskolonnen verwendet werden.

21. Verfahren nach einem oder mehreren der Ansprüche 1 bis 20, **dadurch gekennzeichnet, dass** für die Destillation in Schritt c) Trennwandkolonnen oder thermisch gekoppelte Destillationskolonnen verwendet werden, die als Bodenkolonnen ausgestaltet sind.

22. Verfahren nach einem oder mehreren der Ansprüche 1 bis 21, **dadurch gekennzeichnet, dass** für die Destillation in Schritt c) Trennwandkolonnen oder thermisch gekoppelte Destillationskolonnen verwendet werden, die im Verstärkungsteil mit Packungen, im Abtriebsteil mit Böden ausgestattet sind.

23. Verfahren nach einem oder mehreren der Ansprüche 1 bis 22, **dadurch gekennzeichnet, dass** die Destillation in Schritt c) bei einer Temperatur von 170 bis 260°C und einem Druck von 0,5 bis 500 mbar durchgeführt wird.

24. Verwendung von Trimethylolpropan, das nach einem oder mehreren der Ansprüche 1 bis 23 hergestellt wurde, zur Herstellung von Lackharzen, Pulverlacken, Schaumstoffen oder Polyestern.
